# EUROPÄISCHE PATENTSCHRIFT

(11) **EP 2 018 121 B1**
(45) Veröffentlichungstag und Bekanntmachung des Hinweises auf die Patenterteilung: **29.12.2010**
(21) Anmeldenummer: 07727995.8
(22) Anmeldetag: 11.04.2007
(51) Int. Cl.: A61B 6/14, A61B 6/04

(54) **DENTALES RÖNTGENGERÄT, UMFASSEND EINE AN EINEM TRÄGERTEIL ANGEBRACHTE PATIENTENPOSITIONIERUNG MIT EINER STIRNSTÜTZE**
DENTAL X-RAY APPARATUS COMPRISING A PATIENT-POSITIONING SYSTEM ARRANGED ON A SUPPORT AND PROVIDED WITH A FOREHEAD REST
APPAREIL DE RADIOGRAPHIE DENTAIRE COMPRENANT DES MOYENS DE POSITIONNEMENT D'UN PATIENT AVEC APPUI FRONTAL, APPLIQUÉS SUR UN ÉLÉMENT SUPPORT

(30) Priorität: 08.05.2006 DE 102006021639
(43) Veröffentlichungstag der Anmeldung: 28.01.2009
(73) Patentinhaber: Sirona Dental Systems GmbH, 64625 Bensheim (DE)
(72) Erfinder: STÖCKL, Klaus, 64625 Bensheim (DE)
(74) Vertreter: Sommer, Peter
(86) Internationale Anmeldenummer: PCT/EP2007/053527
(87) Internationale Veröffentlichungsnummer: WO 2007/128644

(56) Entgegenhaltungen:
- EP-A- 0 499 595
- DE-A1- 3 447 862
- DE-A1- 19 844 106
- FR-A- 2 090 727
- FR-A- 2 350 088
- US-A- 3 530 293

## Beschreibung

### Technisches Gebiet

Die Erfindung betrifft ein dentales Röntgengerät, umfassend eine an einem Trägerteil angebrachte Patientenpositionierung mit einer Stirnstütze.

### Stand der Technik

In der DE 3627510 A1 ist eine Kopfpositioniereinrichtung für ein zahnärztliches Röntgendiagnostikgerät zur Erstellung von insbesondere Panorama-Schichtaufnahmen offenbart. Die Einrichtung enthält eine Kinnauflage und eine auch in horizontaler Richtung verstellbar angeordnete und auf den Patientenkopf ausrichtbare Stirnstütze. Der vordere Abschnitt der bügelförmigen Stirnstütze ist an die Stirn des Patientenkopfes anlegbar und an den Kopf des Patienten anpassbar ist. Die Kinnauflage und die Stirnstütze sind an einem Gehäuse angebracht, das mittels eines Trägers an einem Laufwagen schwenkbar befestigt ist, so dass die Kinnauflage und die Stirnstütze sich in ihrer Höhe verstellen lassen. Der Patient blickt beim Anlehnen der Stirn an die Stirnstütze in Richtung des Laufwagens, so dass die Dreheinheit um den Kopf des Patienten gedreht werden kann.

In der DE 36 09 260 A1 ist ein zahnärztliches Röntgendiagnostikgerät zur Erstellung von Panorama-Schichtaufnahmen vom Kiefer eines Patienten offenbart. Zur Positionierung des Patientenkopfes für unterschiedliche Aufnahmearten ist für jede Aufnahmeart ein anderes Positionierglied vorhanden. An einem Träger sind eine Stirnstütze und ein Anlageteil mit einem Aufbeißteil angebracht. Nach der Positionierung des Patientenkopfes blickt der Patient in Richtung eines Laufwagens an einem Ständer.

In der DE 101 53 979 A1 ist ein dentales Röntgengerät mit einer bewegbaren Trägerstruktur für ein zur Erstellung von Röntgenaufnahmen zu bewegendes System offenbart. Zur Positionierung des Patienten sind an der horizontalen Trägerstruktur Haltegriffe und ein Aufbiss vorgesehen. Während der Aufnahme blickt der Patient in Richtung der Trägersäule, während die Trägerstruktur um den Patientenkopf rotiert wird.

In der DE 198 44 106 A1 ist eine Röntgenvorrichtung zum Erzeugen von Panorama-Tomogrammen offenbart, wobei eine Vorrichtung zur Patientenpositionierung an einem Tragarm angeordnet ist, der über einen X/Y-Transportmechanismus gegenüber einer Trägersäule bewegbar ist.

Die DE 34 47 862 A1 offenbart ein Tomographie-Röntgengerät, bei dem eine Vorrichtung zur Patientenpositionierung an einem vertikal verlaufenden Trägerarm angeordnet ist, welcher an einer Tragsäule fest montiert ist, so dass der Patient in bezug auf die Tragsäule transversal positioniert ist.

Nachteilig bei den meisten der genannten Vorrichtungen ist, dass der Patient in Richtung der Trägersäule blickend positioniert wird.

Ein weiterer Nachteil ist, dass die genannten Positionierungseinrichtungen geringen Bewegungsspielraum des Kopfes zulassen.

Die Aufgabe dieser Erfindung besteht daher darin, ein dentales Röntgengerät mit einer Patientenpositionierung bereitzustellen, die eine Positionierung des Kopfes des Patienten im gewünschten Bereich ohne Positionsänderungen während der Aufnahme und eine für den Bediener einfache und übersichtliche Positionierung gewährleistet.

### Darstellung der Erfindung

Diese Aufgabe wird durch die Merkmale des Hauptanspruchs 1 gelöst.

Erfindungsgemäß wird ein dentales Röntgengerät vorgeschlagen, welches eine an einem Trägerteil angebrachte Patientenpositionierung mit einer Stirnstütze umfasst. Dabei ist an dem Trägerteil ein horizontal schwenkbarer Tragarm angeordnet, an dem wiederum die Stirnstütze angeordnet ist.

Dadurch kann der Tragarm nach dem Platzieren des Patienten zum Patienten hin horizontal geschwenkt werden, um den Patientenkopf mittels der Stirnstütze zu positionieren. Der Patient blickt dabei aus dem Gerät heraus.

Der Tragarm weist ein Schwenkgelenk auf, wobei das Schwenkgelenk zum Trägerteil beabstandet seitlich neben einer Patientenposition angeordnet ist. Der Tragarm ist in einer Aufnahmeposition bogenförmig um den Patienten herumführt und endet im Bereich des Kinns des Patienten, wobei in einer Einstiegsposition zumindest ein Teil des Tragarms von der Patientenposition entfernt angeordnet ist.

Dadurch kann der Patient mit der Rücken zum Trägerteil positioniert werden, wobei der Tragarm bogenförmig um den Patienten geführt ist und an dessen Kinn endet. Die Positionierung wird für den Bediener erleichtert, da er freie Sicht auf die zu positionierende Vorderseite des Patientenkopfes hat.

Vorteilhafterweise kann das Trägerteil höhenverstellbar sein und es kann an dem Trägerteil ein Röntgenstrahler und ein Bildempfänger angebracht sein.

Dadurch kann das Trägerteil in seiner Höhenlage an die Körpergröße des Patienten angepasst werden, so dass der Röntgenstrahler und der Bildempfänger in horizontaler Ebene um den aufzunehmenden Bereich des Patienten rotiert werden kann.

Vorteilhafterweise kann am Tragarm ein Aufbiss angeordnet sein.

Dadurch wird zusätzlich zur Stirnstütze ein weiteres Positionierungsmittel zur Verfügung gestellt, so dass eine genauere Positionierung ermöglicht wird.

Vorteilhafterweise kann das Schwenkgelenk ein Rastmittel aufweisen, welches mit einem Schwenkhebel am Schwenkarm derart zusammenwirkt, dass der Schwenkhebel in einer Aufnahmeposition des Tragarms einen Kraftschluss herstellt.

Dadurch wird der Tragarm mit der Stirnstütze in der Aufnahmeposition fixiert, so dass der Patientenkopf während der Aufnahme keine Positionsänderung erfährt.

Vorteilhafterweise kann der Schwenkhebel federbelastet sein und der Tragarm eine Entriegelungsvorrichtung zum Lösen des Kraftschlusses aufweisen.

Dadurch kann der Tragarm nach der Röntgenaufnahme entriegelt werden und aus der Aufnahmeposition geschwenkt werden, so dass der Patient aussteigen kann.

Vorteilhafterweise kann die Entriegelungsvorrichtung einen am Ende des Tragarms angeordneten Drehgriff mit einem Übertragungsmittel zu einem Entriegelungsteil für den Schwenkhebel aufweisen.

Dadurch kann der Tragarm bequem durch das Betätigen des Drehgriffs entriegelt werden.

Vorteilhafterweise kann das Übertragungsmittel eine Welle und das Entriegelungsteil ein Exzenter sein. Auf der Welle kann zusätzlich noch mindestens ein Exzenter vorgesehen sein, die mit einem Führungsmittel für eine Stirnstütze zum Zweck des Vorschwenkens zusammenwirkt.

Die Verwendung eines Exzenters als Entriegelungsteil ist eine zuverlässige und einfach zu realisierende technische Lösung, so dass der Wartungs- und Produktionsaufwand vermindert wird.

Vorteilhafterweise kann der Exzenter für das Zusammenwirken mit dem Führungsmittel und der Exzenter für die Entriegelung des Schwenkhebels auf einer gemeinsamen Welle angeordnet sein.

Dadurch werden die Entriegelung auslösenden Drehkräfte auf eine einfache und zuverlässige Weise übertragen.

Vorteilhafterweise kann mit dem Drehgriff durch eine Drehbewegung in dieselbe Richtung sowohl die Stirnstütze von der Patientenposition weg verstellbar als auch der Schwenkhebel entriegelbar sein.

Dadurch werden zwei Funktionen durch den Drehgriff gesteuert und die Bedienung vereinfacht.

Vorteilhafterweise können die Exzenter so an der Welle angeordnet sein, dass ausgehend von einer Patientenposition beim Drehen des Drehgriffs zunächst die Stirnstütze in eine patientenferne Ausgangsstellung gebracht ist und erst bei weiterem Drehen die Entriegelung des Schwenkhebels erfolgt, wozu die Exzenter einen im wesentlichen überschneidungsfreien Winkelbereich aufweisen.

Dadurch wird durch eine Drehbewegung zunächst die Stirnstütze von der Stirn des Patienten entfernt und anschließend wird der Schwenkhebel entriegelt.

Vorteilhafterweise kann die Stirnstütze mindestens einen Stab umfassen, der in das mindestens eine Führungsmittel einsteckbar ist und dort höhenverstellbar geführt ist. Weiterhin kann ein Anlageteil mit dem Stab verbunden sein.

Dadurch kann die Stirnstütze durch das Verstellen des Stabs individuell an die Kopfgröße des Patienten individuell angepasst werden.

Vorteilhafterweise kann die Stirnstütze mindestens einen Stab umfassen, der in das mindestens eine Führungsmittel eingesteckt ist, wobei ein gegenüber dem Stab höhenverstellbar geführtes Anlageteil vorgesehen ist.

Dadurch kann die Stirnstütze individuell an die Kopfgröße des Patienten angepasst werden, indem das Anlageteil in seiner Höhe gegenüber dem Stab höhenverstellt wird.

Vorteilhafterweise kann am Tragarm ein weiterer Schwenkarm für ein Bedienpaneel angeordnet sein.

Dadurch befindet sich das Bedienpaneel und die Patientenpositionierung im Blickfeld des Bedieners, so dass eine Positionierung ohne die Blickrichtung zu wechseln möglich ist.

Vorteilhafterweise kann der weitere Schwenkarm am Schwenkgelenk schwenkbar befestigt sein.

Dadurch wird ein weiterer unabhängig schwenkbarer Schwenkarm bereitgestellt, an dem beispielweise weitere Positionierungsmittel oder das Bedienpaneel angebracht werden können.

Vorteilhafterweise können am Tragarm Haltegriffe angeordnet sein.

Dadurch kann der Patient sich an den Haltegriffen festhalten, so dass der Patient während der Röntgenaufnahme in seiner Position unverändert bleibt. Insbesondere bei stehendem Patient ist die zusätzliche Orientierung an den Haltegriffen von Vorteil.

Kurzbeschreibung der Zeichnung Ausführungsbeispiele der Erfindung sind in der Zeichnung dargestellt. Es zeigt die
- Fig.1: ein dentales Röntgengerät mit einem Schwenkarm;
- Fig. 2: eine Seitenansicht des dentalen Röntgengeräts aus Fig. 1 dargestellt
- Fig.3: eine Stirnstütze und der Aufbiss am Ende des Schwenkarms;
- Fig. 4: das Schwenkgelenk und die Drehung der Stirnstütze;
- Fig. 5: das Zusammenwirken der Zahnscheibe mit dem Schwenk- hebel im Detail;
- Fig. 6: der Schwenkmechanismus der Stirnstütze im Detail;
- Fig. 7: die schematische Aufteilung des Verdrehwinkels des Drehgriffs;
- Fig. 8: ein zweiter am Tragarm angeordneter Schwenkarm für ein Bedienpaneel.

### Ausführungsbeispiel der Erfindung

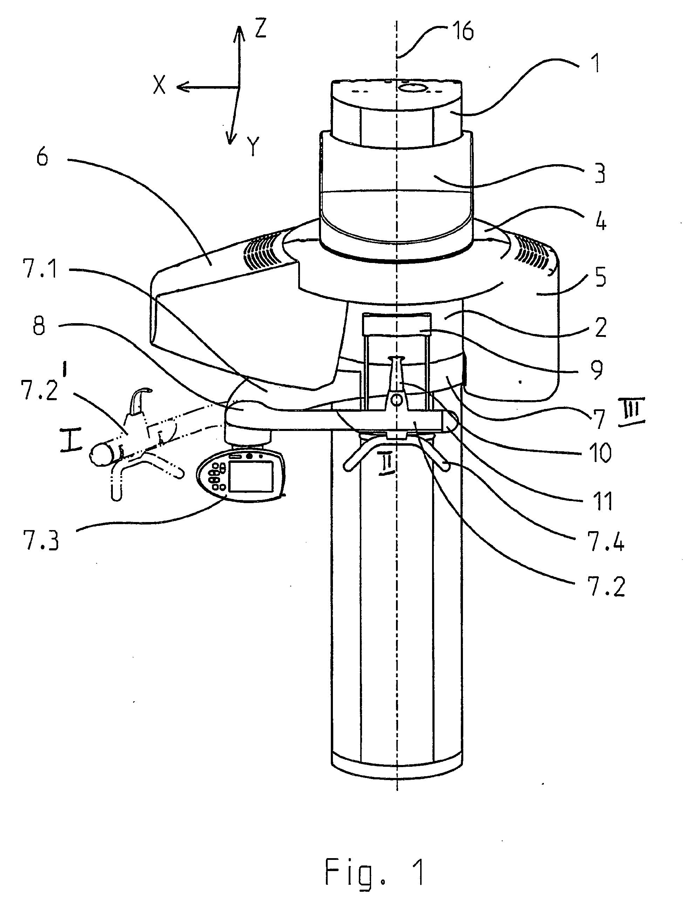

In Fig. 1 ist ein dentales Röntgengerät dargestellt. An einer Säule 1 ist ein Trägerteil 2 höhenverstellbar entlang einer Achse 16 geführt. An dem Trägerteil 2 ist ein Ausleger 3 befestigt, an dem eine Dreheinheit 4 mit einem daran befestigten Röntgenstrahler 5 und einem Bildempfänger 6 angeordnet ist. Die Dreheinheit 4 ist so ausgebildet, dass ein nicht dargestellter Patientenkopf zwischen dem Röntgenstrahler 5 und dem Bildempfänger 6 zumindest ein Stück weit bogenförmig umfahren werden kann.

Mit dem Trägerteil 2 ist ein Tragarm 7 verbunden, der zusammen mit dem Trägerteil 2 höhenverstellbar ist. Der Tragarm 7 weist ein Schwenkgelenk 8 auf, das den Tragarm 7 in einen feststehenden Tragarmabschnitt 7.1 und in einen Schwenkarm 7.2 unterteilt. Der Schwenkarm 7.2 lässt sich um das Schwenkgelenk 8 in die gestrichelt eingezeichnet Einstiegsposition für einen nicht dargestellten Patienten bringen, wobei der Schwenkarm dann mit 7.2' bezeichnet ist. In der Aufnahmeposition umschließt der Schwenkarm 7.2 den Patienten zumindest teilweise, da das Schwenkgelenk 8 seitlich von dem Patienten in einem ersten seitlichen Bereich I angeordnet ist.

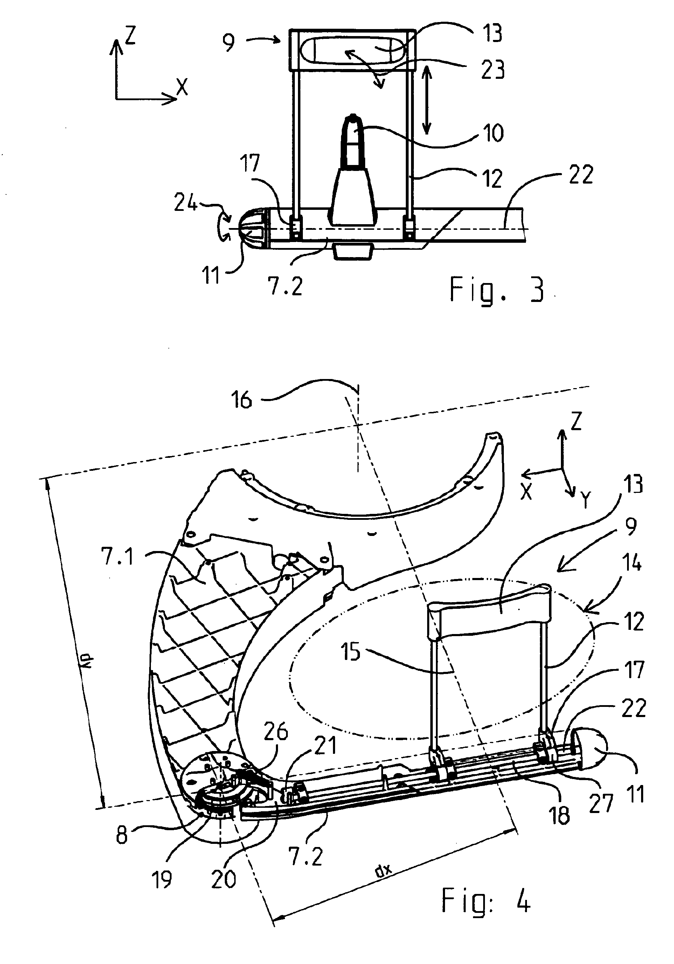

An dem Schwenkarm 7.2 ist eine Stirnstütze 9 und vorzugsweise auch ein Aufbiss 10 angeordnet, um den Patienten während der Aufnahme in seiner Position zu fixieren. Am Ende des Tragarms befindet sich ein Drehgriff 11 als Teil einer Verriegelungseinheit für das Schwenkgelenk 8, dessen Funktion in Fig. 4 näher beschrieben wird.

Der Schwenkarm 7.2 umfasst den Patienten und endet mit dem Drehgriff 11 zwischen einem mittleren Bereich II und einem zweiten seitlichen Bereich III, so dass der Aufbiss 10 am Schwenkarm 7.2 in einer Aufnahmeposition im mittleren Bereich II angeordnet werden kann.

Am Schwenkgelenk 8 ist an der Unterseite ein Bedienpaneel 7.3 angeordnet, das eine Bedienung des dentalen Röntgengeräts durch einen Bediener ermöglicht.

Weiterhin sind im Bereich des Aufbisses 10 an der Unterseite des Schwenkarms 7.2 Haltegriffe 7.4 angeordnet, die dem Patienten ermöglichen sich während der Röntgenaufnahme festzuhalten, so dass seine Position zusätzlich zur Fixierung durch den Aufbiss 10 fixiert wird.

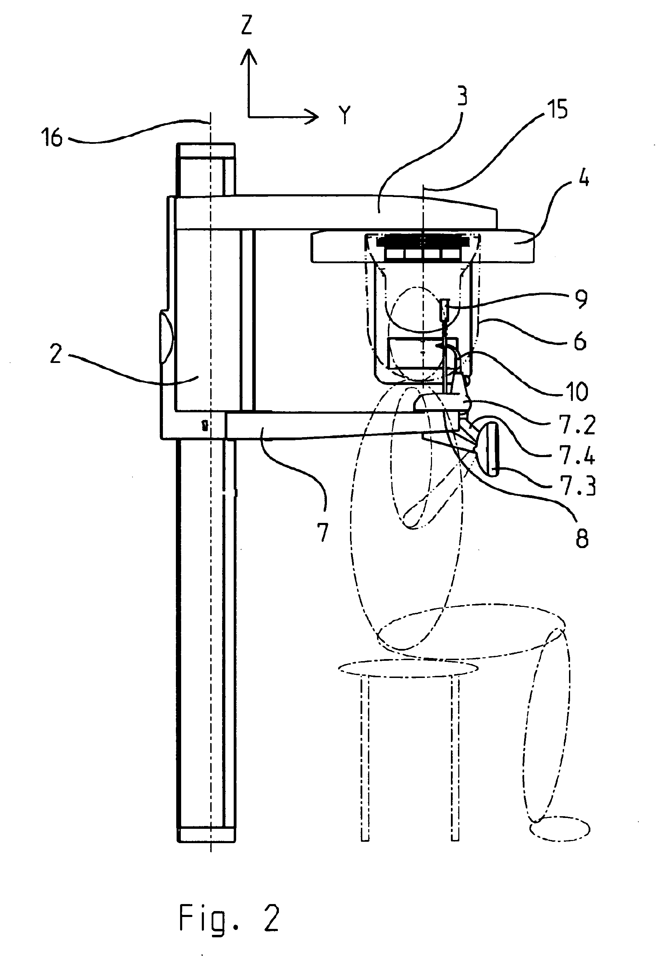

In Fig. 2 ist eine Seitenansicht aus Fig. 1 dargestellt, wobei wieder die Säule 1 mit dem vertikal entlang der Achse 16 geführten Trägerteil 2 zu erkennen ist. An dem Trägerteil 2 ist der Ausleger 3 mit der Dreheinheit 4 gezeigt, wobei in der dargestellten Ansicht auf den an der Dreheinheit 4 angebrachten Empfänger 6 geschaut wird, der den Röntgenstrahler 5 (Fig. 1) und einen Patientenkopf sowie die Stirnstütze 9 und den Aufbiss 10 teilweise verdeckt.

Die Dreheinheit 4 dreht sich um die Achse 15, die parallel zu der Achse 16 und in einem Abstand zu dieser angeordnet ist.

An dem Trägerteil 2 ist wiederum der Tragarm 7 mit dem Schwenkgelenk 8 und dem Schwenkarm 7.2 zu erkennen. An der Unterseite des Schwenkgelenks ist das Bedienpaneel 7.3 und am Schwenkarm 7.2 die Haltegriffe 7.4 zu erkennen. Der schematisch dargestellte Patient ist in einer Sitzposition gezeigt.

In Fig. 3 ist die Stirnstütze 9 und der Aufbiss 10 am Ende des Schwenkarms 7.2 im Detail dargestellt. Die Ansicht zeigt den Blick vom Patienten ausgesehen, also von der Achse 16 zur Achse 15 in y-Richtung (Fig. 2). Die Stirnstütze 9 umfasst zwei in einem Abstand zueinander an dem Schwenkarm 7.2 angeordnete Stäbe 12, an deren Enden ein Anlageteil 13 vorgesehen ist. Die Stäbe 12 sind in einem Führungsmittel 17 höhenverstellbar geführt, wobei sich die Führungsmittel 17 und zusammen mit diesen auch die Stäbe 12 und das Anlageteil 13 um eine Drehachse 22 in Richtung des Pfeils 23 zum Patienten hin und von diesem weg verdrehen lassen. Dies geschieht durch Betätigung des Drehgriffes 11 in Richtung des Pfeils 24.

In Fig. 4 ist das Schwenkgelenk 8 einerseits und die Drehung der Stirnstütze 9 andererseits exemplarisch dargestellt. Der feststehende Tragarmabschnitt 7.1 erstreckt sich bis zu dem Schwenkgelenk 8, so dass sich ein Abstand dy zum Trägerteil ergibt. Auf Grund der seitlichen Anordnung des Schwenkgelenks 8 bezüglich der Patientenposition 14 und der Achse 15 weist das Schwenkgelenk 8 auch einen Abstand dx auf.

Das Schwenkgelenk 8 umfasst eine Zahnscheibe 19, die mit dem Tragarmabschnitt 7.1 starr verbunden ist und die mit einem Schwenkhebel 20 am Schwenkarm 7.2 zusammenwirkt. Der Schwenkarm 7.2 ist zwischen der Zahnscheibe 19 und dem Tragarmabschnitt 7.1 drehbar gelagert.

Der Schwenkhebel 20 wird von einer Feder 26 in die Zahnscheibe 19 gedrückt, wodurch ein Kraftschluss bzw. Formschluss hergestellt ist. Der Schwenkhebel 20 wird über ein Exzenter 21 gegen die Federkraft 26 aus der Verzahnung der Zahnscheibe 19 gedrückt, wodurch der Kraftschluss bzw. Formschluss aufgehoben wird. Dazu ist der Exzenter 21 an einer Welle 18 angeordnet, die durch den Drehgriff 11 verdreht wird.

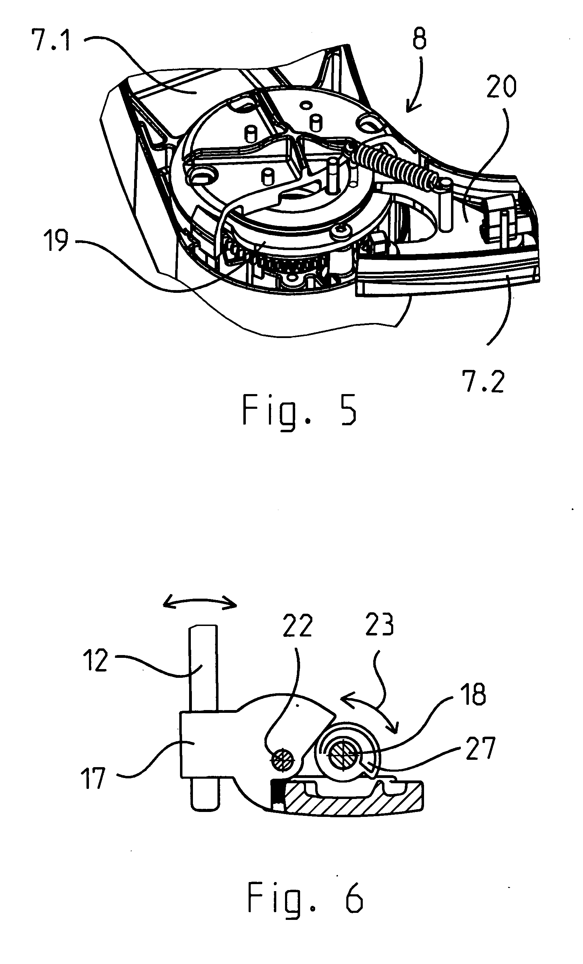

Am Schwenkarm 7.2 ist darüber hinaus die Stirnstütze 9 angeordnet, wobei deren Führungsmittel 17 für die Stäbe 12, welche das Anlageteil 13 tragen, mit jeweils einem Exzenter 27 zusammenwirken. Der Exzenter 27 ist mit der Welle 18 drehfest verbunden und beim Verdrehen der Welle 18 über den Drehgriff 11 wird das Führungsmittel 17 um die Drehachse 22 verschwenkt, die parallel zur Welle 18 ist. Die Drehachse ist in Fig. 6 dargestellt.

In Fig. 5 ist das Zusammenwirken der Zahnscheibe 19 mit dem Schwenkhebel 20 im Detail erläutert. Der Tragarmabschnitt 7.1 trägt die starr verbundene Zahnscheibe 19 und der Schwenkarm 7.2 ist dazwischenliegend angeordnet und gelagert. An dem Schwenkarm 7.2 ist darüber hinaus der Schwenkhebel 20 drehbar gelagert, wobei in der gezeigten Stellung der Schwenkhebel 20 mit der Zahnscheibe 19 in Eingriff und folglich ein Kraftschluss bzw. Formschluss hergestellt ist.

In Fig. 6 ist der Schwenkmechanismus der Stirnstütze im Detail gezeigt. Das Führungsmittel 17 ist um eine Welle 22 drehbar gelagert und liegt an einem Ende an dem mit der Welle 18 drehfest verbundenen Exzenter 27 an. Beim Verdrehen des Exzenters 27 durch Drehung der Welle 18 mittels des nicht dargestellten Drehgriffes wird der Exzenter 27 in Richtung des Pfeils 23 verdreht, so dass der Stab 12 entweder zum Patienten hin oder von diesem weg um die Achse 22 geschwenkt wird. Dies geschieht dadurch, dass eine Anlagefläche des Führungsmittels 17 an dem Exzenter 27 geführt wird. Die Rückholung des Führungsmittels 17 geschieht beispielsweise mittels einer nicht dargestellten Rückholfeder.

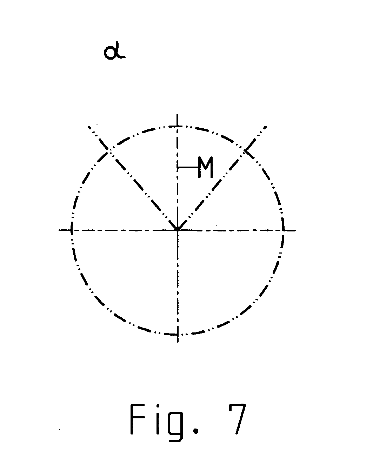

In Fig. 7 ist die Aufteilung des Verdrehwinkels des Drehgriffs 11 aus Fig. 1 schematisch dargestellt. In einem ersten Winkelbereich α wird ausgehend von einer Mittelposition M bei der Einstiegsposition des Schwenkarms 7.2 aus Fig. 1, also etwa bei geöffnetem Schwenkarm 7.2 oder bei gerade geschlossenem Schwenkarm 7.2 durch ein Drehen des Drehgriffs vorzugsweise in Richtung Patient die Stirnstütze 9 aus Fig. in Richtung Patient geschwenkt, und die Stirnstütze 9 zur Anlage an die Stirn des Patientenkopfes gebracht. Der Winkelbereich α ist so bemessen, dass er in der Praxis nur in Extremfällen ausgeschöpft wird. In der Regel ist nur ein teilweises Verschwenken innerhalb des Winkelbereichs α ausreichend, um die Stirn des Patienten an der gewünschten Position zu fixieren. Durch ein Drehen des Drehgriffes 11 in die entgegengesetzte Richtung wird die Stirnstütze 9 weg vom Patienten bis zu einer senkrechten Endstellung geschwenkt.

Der zweite Winkelbereich β schließt ohne Überlappung an den Winkelbereich α an, wobei auch ein dazwischenliegender Winkelbereich vorhanden sein kann. Beim Verdrehen über den Winkelbereich β wird die Verriegelung des Schwenkgelenks 8 aus Fig. 1 gelöst.

Wird der Drehgriff weiter in den Winkelbereich β hineingedreht, wird der Schwenkhebel 20 aus Fig. 4 über den mit der Welle 18 aus Fig. 4 verbundenen Exzenter 21 aus Fig. 4 aus der eingerasteten Position herausgeführt und das Schwenkgelenk 8 wird freigegeben, so dass der Schwenkarm 7.2 aus seiner Aufnahmeposition in eine Einstiegsposition herausgeschwenkt werden kann, um dem Patienten den Ein- und Ausstieg aus dem Gerät zu ermöglichen.

Mit einem einzigen Bedienelement in Form des Drehgriffs 11 kann also sowohl die Einstellung der Stirnstütze 9 als auch das Entriegeln des Schwenkarms 7.2 erfolgen. Vor der Entriegelung des Schwenkarms 7.2 wird darüber hinaus die Stirnstütze 9 in eine patientenferne Ausgangstellung gebracht.

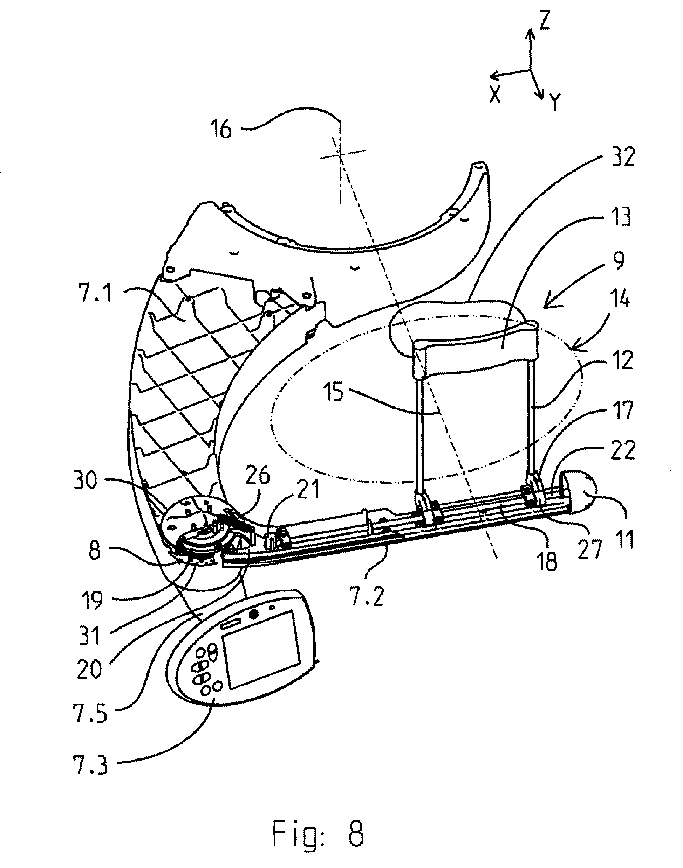

In Fig. 8 ist dargestellt, dass am Tragarm 7.1 neben dem Schwenkarm 7.2 ein weiterer Schwenkarm 7.5 für das Bedienpaneel 7.3 angeordnet sein kann. Der weitere Schwenkarm 7.5 kann dann am Schwenkgelenk 8 schwenkbar befestigt sein. Dazu kann das Schwenkgelenk 8 eine obere Lagerung 30 und eine untere Lagerung 31 aufweisen, an denen der jeweilige Schwenkarm angebracht ist. Dabei kann der Schwenkarm 7.2 für die Stirnstütze 9 über dem Schwenkarm 7.5 für das Bedienpaneel angeordnet sein.

Darüber hinaus besteht die Möglichkeit, die Schwenkbewegung des Schwenkarms 7.2 mit der Schwenkbewegung des Schwenkarms 7.5 für das Bedienpaneel zu koppeln, etwa um beim Öffnen des Schwenkarms 7.2 auch das Bedienpaneel von dem Patientenbereich wegzubewegen. Dies kann beispielsweise durch einen einfachen Mitnehmer am Schwenkarm 7.2 oder im Schwenkgelenk geschehen.

Um Patienten, denen es schwer fällt ruhig zu halten einem noch besseren Halt zu geben, kann darüber hinaus ein elastisches Band 32 vorgesehen werden, das um den Kopf des Patienten gelegt wird und an der Stirnstütze 9 an entsprechenden Haken mit leichter Spannung eingehängt wird.

### Bezugszeichenliste

- 1: Säule
- 2: Trägerteil
- 3: Ausleger
- 4: Dreheinheit
- 5: Röntgenstrahler
- 6: Bildempfänger
- 7: Tragarm
- 7.1: Tragarmabschnitt
- 7.2: Schwenkarm
- 7.2': Schwenkarm in der Einstiegsposition
- 7.3: Bedienpaneel
- 7.4: Haltegriffe
- 7.5: Schwenkarm
- 8: Schwenkgelenk
- 9: Stirnstütze
- 10: Aufbiss
- 11: Drehgriff
- 12: Stäbe
- 13: Anlageteil
- 14: Patientenposition
- 15: Achse der Dreheinheit 4
- 16: Achse des Trägerteils 2
- 17: Führungsmittel
- 18: Welle
- 19: Zahnscheibe
- 20: Schwenkhebel
- 21: Exzenter
- 22: Drehachse
- 23: Pfeil
- 24: Pfeil
- 25:
- 26: Feder
- 27: Exzenter
- 30: obere Lagerung
- 31: untere Lagerung
- 32: elastisches Band
- I: erster seitlicher Bereich
- II: mittlerer Bereich
- III: zweiter seitlicher Bereich

## Patentansprüche

1. Dentales Röntgengerät, aufweisend ein Trägerteil (2) und eine Stirnstütze (9) zur Patientenpositionierung wobei an dem Trägerteil (2) ein horizontal schwenkbarer Tragarm (7) angeordnet ist, an dem wiederum die Stirnstütze (9) angeordnet ist **dadurch gekennzeichnet, dass** der Tragarm (7) ein Schwenkgelenk (8) aufweist, wobei das Schwenkgelenk (8) zum Trägerteil (2) beabstandet und in einem ersten seitlichen Bereich (I) neben einer Patientenposition (14) angeordnet ist und wobei der Tragarm (7) in einer Aufnahmeposition bogenförmig um die Patientenposition herumführt und über einen mittleren Bereich (II) der Patientenposition hinausreicht.

2. Röntgengerät nach Anspruch 1, **dadurch gekennzeichnet, dass** das Trägerteil (2) höhenverstellbar ist und dass an dem Trägerteil (2) ein Röntgenstrahler (5) und ein Bildempfänger (6) angebracht sind.

3. Röntgengerät nach Anspruch 1 oder 2, **dadurch gekennzeichnet, dass** der Tragarm (7) vor einem dem ersten seitlichen Bereich (I) gegenüberliegenden zweiten seitlichen Bereich (III) der Patientenposition (14) endet, wobei ein Teil des Tragarms (7) einen Schwenkarm (7.2) bildet, der in einer Einstiegsposition (7.2') gegenüber der Patientenposition (14) entfernt angeordnet ist.

4. Röntgengerät nach einem der Ansprüche 1 bis 3, **dadurch gekennzeichnet, dass** das Schwenkgelenk (8) einen Verriegelungsmechanismus aufweist.

5. Röntgengerät nach Anspruch 4, **dadurch gekennzeichnet, dass** das Verriegelungsmechanismus ein Rastmittel (19) aufweist, welches mit einem Schwenkhebel (20) am Tragarm (7) derart zusammenwirkt, dass der Schwenkhebel (20) in einer Aufnahmeposition des Tragarms (7) einen Kraftschluss bzw. Formschluss herstellt.

6. Röntgengerät nach Anspruch 4 oder 5, **dadurch gekennzeichnet, dass** der Tragarm (7) eine Entriegelungsvorrichtung zum Lösen des Kraftschlusses aufweist.

7. Röntgengerät nach Anspruch 6, **dadurch gekennzeichnet, dass** die Entriegelungsvorrichtung einen am Ende des Tragarms (7) angeordneten Drehgriff (11) aufweist.

8. Röntgengerät nach Anspruch 7, **dadurch gekennzeichnet, dass** die Entriegelungsvorrichtung ein Übertragungsmittel zu einem Entriegelungsteil für den Schwenkhebel (20) aufweist.

9. Röntgengerät nach Anspruch 8, **dadurch gekennzeichnet, dass** das Übertragungsmittel eine Welle (18) und das Entriegelungsteil ein Exzenter (21) ist.

10. Röntgengerät nach Anspruch 9, **dadurch gekennzeichnet, dass** auf der Welle (18) zusätzlich noch mindestens ein Exzenter (27) vorgesehen ist, der mit einem Führungsmittel für eine Stirnstütze (9) zum Zweck des Vorschwenkens der Stirnstütze (9) zusammenwirkt.

11. Röntgengerät nach Anspruch 10, **dadurch gekennzeichnet, dass** mit dem Drehgriff (11) durch eine Drehbewegung in dieselbe Richtung sowohl die Stirnstütze (9) von der Patientenposition (14) weg verstellbar als auch der Schwenkhebel (20) entriegelbar ist.

12. Röntgengerät nach Anspruch 11, **dadurch gekennzeichnet, dass** die Exzenter (21, 27) so an der Welle (18) angeordnet sind, dass ausgehend von einer Patientenposition (14) beim Drehen des Drehgriffs (11) zunächst die Stirnstütze (9) in eine patientenferne Ausgangsstellung gebracht ist und erst bei weiterem Drehen die Entriegelung des Schwenkhebels (20) erfolgt, wozu die Exzenter (21, 27) einen im wesentlichen überschneidungsfreien Winkelbereich (α, β) aufweisen.

13. Röntgengerät nach einem der Ansprüche 9 bis 12, **dadurch gekennzeichnet, dass** die Stirnstütze (9) mindestens einen Stab (12) umfasst, der in das mindestens eine Führungsmittel (17) einsteckbar ist und dort höhenverstellbar geführt ist und dass weiterhin ein Anlageteil (13) mit dem Stab (12) verbunden ist.

14. Röntgengerät nach einem der Ansprüche 9 bis 13, **dadurch gekennzeichnet, dass** die Stirnstütze (9) mindestens einen Stab (12) umfasst, der in das mindestens eine Führungsmittel (17) eingesteckt ist und dass ein gegenüber dem Stab (12) höhenverstellbar geführtes Anlageteil (13) vorgesehen ist.

## Claims

1. A dental X-ray apparatus, comprising a carrying member (2) and a forehead rest (9) for patient-positioning, wherein on said carrying member (2) there is disposed a horizontally pivotable carrying arm (7) on which, in turn, said forehead rest (9) is disposed, **characterised in that** said carrying arm (7) comprises a swivel joint (8), and said swivel joint (8) is set at a distance from said carrying member (2) and is disposed in a first lateral region (I) next to the patient's position (14), and that said carrying arm (7), in an imaging position, passes around the patient's position in an arcuate manner and extends beyond a central region (II) of the patient's position.

2. The X-ray apparatus as defined in claim 1, **characterized in that** said carrying member (2) is vertically adjustable and that an X-ray emitter (5) and an image detector (6) are mounted on said carrying member (2).

3. The X-ray apparatus as defined in claim 1 or claim 2, **characterized in** said carrying arm (7) terminates at the commencement of a second lateral region (III) of said patient's position (14) opposite said first lateral region (I), and a part of said carrying arm (7) forms a swivel arm (7.2) which, in an entry position (7.2'), is disposed at a distance from said patient's position (14).

4. The X-ray apparatus as defined in any one of claims 1 to 3, **characterized in that** said swivel joint (8) comprises a locking mechanism.

5. The X-ray apparatus as defined in claim 4, **characterized in that** said locking mechanism has locking means (19) cooperating with a pivoted lever (20) on said carrying arm (7) such that said pivoted lever (20) creates, in an imaging position of said carrying arm (7), a frictional connection or positive fit.

6. The X-ray apparatus as defined in claim 4 or claim 5, **characterized in that** said carrying arm (7) has an unlocking device for disconnecting said frictional connection.

7. The X-ray apparatus as defined in claim 6, **characterized in that** said unlocking device has a rotary handle (11) disposed at the end of said carrying arm (7).

8. The X-ray apparatus as defined in claim 7, **characterized in that** said unlocking device has transmission device extending to said unlocking member for said pivoted lever (20).

9. The X-ray apparatus as defined in claim 8, **characterized in that** said transmission device is a shaft (18) and said unlocking member is an excentric (21).

10. The X-ray apparatus as defined in claim 9, **characterized in that** on said shaft (18) there is additionally provided at least one further excentric (27) which cooperates with guide means for a forehead rest (9) for the purpose of swinging said forehead rest (9) forward.

11. The X-ray apparatus as defined in claim 10, **characterized in that** said rotary handle (11) is adapted, when rotated in one specific direction, both to move said forehead rest (9) away from said patient's position (14) and to unlock said pivoted lever (20).

12. The X-ray apparatus as defined in claim 11, **characterized in that** said excentrics (21, 27) are disposed on said shaft (18) such that, starting from a patient's position (14), rotation of said rotary handle (11) first of all moves said forehead rest (9) to a home position at a distance from said patient and then, on further rotation, unlocks said pivoted lever (20), to which end said excentrics (21, 27) cover a non-overlapping angular range (α, β).

13. The X-ray apparatus as defined in any one of claims 9 to 12, **characterized in that** said forehead rest (9) has at least one bar (12) which can be pushed into the at least one guide means (17), where it is guided for vertical adjustment, and also that a rest (13) is connected to said bar (12).

14. The X-ray apparatus as defined in any one of claims 9 to 13, **characterized in that** said forehead rest (9) comprises at least one bar (12), which is inserted into said at least one guide means (17) and that a rest (13) is provided which is vertically adjustable relatively to said bar (12).

## Revendications

1. Appareil d'imagerie dentaire, présentant une pièce de support (2) et un support frontal (9) pour le positionnement du patient, dans lequel un bras porteur (7) horizontal pivotant est placé sur la pièce de support (2), sur lequel bras le support frontal (9) est placé à son tour, **caractérisé en ce que** le bras porteur (7) présente une articulation pivotante (8), sachant que l'articulation pivotante (8) est distante de la pièce de support (2) et est placée dans une première zone latérale (I) près d'une position du patient (14) et sachant que le bras porteur (7) tourne autour de la position du patient en formant un arc de cercle dans une position de prise de vue et fait saillie au-dessus d'une zone centrale (II) de la position du patient.

2. Appareil d'imagerie dentaire selon la revendication 1, **caractérisé en ce que** la pièce de support (2) est réglable en hauteur et qu'un émetteur radiologique (5) et un récepteur d'images (6) sont placés sur la pièce de support (2).

3. Appareil d'imagerie dentaire selon la revendication 1 ou 2, **caractérisé en ce que** le bras porteur (7) termine devant une deuxième zone latérale (III) de la position du patient (14) qui fait face à la première zone latérale (I), sachant qu'une partie du bras porteur (7) forme un bras pivotant (7.2), lequel, dans une position de montée (7.2'), est éloigné par rapport à la position du patient (14).

4. Appareil d'imagerie dentaire selon l'une des revendications 1 à 3, **caractérisé en ce que** l'articulation pivotante (8) présente un mécanisme de verrouillage.

5. Appareil d'imagerie dentaire selon la revendication 4, **caractérisé en ce que** le mécanisme de verrouillage présente un moyen d'encliquetage (19) qui coopère avec un levier pivotant (20) sur le bras porteur (7) de telle manière que le levier pivotant (20) crée une complémentarité des formes respectivement une adhérence des forces dans une position de prise de vue du bras porteur (7).

6. Appareil d'imagerie dentaire selon la revendication 4 ou 5, **caractérisé en ce que** le bras porteur (7) présente un dispositif de déverrouillage pour relâcher l'adhérence des forces.

7. Appareil d'imagerie dentaire selon la revendication 6, **caractérisé en ce que** le dispositif de déverrouillage présente une poignée tournante (11) placée à l'extrémité du bras porteur (7).

8. Appareil d'imagerie dentaire selon la revendication 7, **caractérisé en ce que** le dispositif de déverrouillage présente un moyen de transmission vers une pièce de déverrouillage pour le levier pivotant (20).

9. Appareil d'imagerie dentaire selon la revendication 8, **caractérisé en ce que** le moyen de transmission est un arbre (18) et que la pièce de déverrouillage est un excentrique (21).

10. Appareil d'imagerie dentaire selon la revendication 9, **caractérisé en ce qu'**au moins un excentrique (27) est prévu en plus sur l'arbre (18) et coopère avec un moyen de guidage pour un support frontal (9) pour faire pivoter le support frontal (9) vers l'avant.

11. Appareil d'imagerie dentaire selon la revendication 10, **caractérisé en ce qu'**avec la poignée rotative (11), par une rotation dans le même sens, tant le support frontal (9) peut être éloigné de la position du patient (14) que le levier pivotant (20) peut être déverrouillé.

12. Appareil d'imagerie dentaire selon la revendication 11, **caractérisé en ce que** les excentriques (21, 27) sont ainsi placés sur l'arbre (18) qu'en partant d'une position du patient (14) lorsque l'on tourne la poignée tournante (11), le support frontal (9) est d'abord amené dans une position de départ éloignée du patient et seulement lorsque l'on tourne encore, le levier pivotant (20) se déverrouille, ce pour quoi les excentriques (21, 27) présentent une zone angulaire essentiellement libre de chevauchements (α, β).

13. Appareil d'imagerie dentaire selon l'une des revendications 9 à 12, **caractérisé en ce que** le support frontal (9) comprend au moins une baguette (12) qui peut être insérée dans l'au moins un moyen de guidage (17) et qui y est dirigée en étant réglable en hauteur et qu'en outre, une pièce d'appui (13) est reliée à la baguette (12).

14. Appareil d'imagerie dentaire selon l'une des revendications 9 à 13, **caractérisé en ce que** le support frontal (9) comprend au moins une baguette (12) qui est insérée dans l'au moins un moyen de guidage (17) et qu'une pièce d'appui (13) réglable en hauteur par rapport à la baguette (12) est prévue.
